# EUROPEAN PATENT APPLICATION

(11) **EP 4 830 742 A1**
(43) Date of publication of application: **28.10.2026**
(21) Application number: 24905725.8
(22) Date of filing: 27.09.2024
(51) Int. Cl.: A61M 1/00, A61B 17/22

(54) **INTERMITTENT SUCTION THROMBUS ASPIRATION PUMP SYSTEM**

(30) Priority: 22.12.2023 CN 202311786227
(71) Applicant: Curatia Medical Limited, Suzhou, Jiangsu 215011 (CN)
(72) Inventor: JIN, Hu, Suzhou, Jiangsu 215011 (CN); SUN, Jianguo, Suzhou, Jiangsu 215011 (CN); CHIU, Jessica, Suzhou, Jiangsu 215011 (CN)
(74) Representative: Metida
(86) International application number: PCT/CN2024/121602
(87) International publication number: WO 2025/130235

(57) **Abstract**

An intermittent suction thrombus aspiration pump system includes: a suction catheter, a flow resistor, a first pressure sensor, a second pressure sensor, a metering pump, a clamp valve, a collection container, a third pressure sensor, a negative pressure regulating valve, and a negative pressure pump. The suction catheter, the first pressure sensor, the flow resistor, the second pressure sensor, the clamp valve, the collection container, the third pressure sensor, the negative pressure regulating valve, and the negative pressure pump are connected in sequence. Two ends of the metering pump are respectively connected to two ends of the clamp valve. The intermittent suction thrombus aspiration pump system can actively increase the pressure difference without increasing the negative pressure output by the negative pressure pump, thereby achieving the purpose of instantly sucking thrombus.

## Description

### TECHNICAL FIELD

The present invention relates to the technical field of intravascular thrombus aspiration, and in particular to an intermittent suction thrombus aspiration pump system.

### BACKGROUND

Thrombosis is a common clinical disease. The main hazards of thrombus are as follows: 1) thrombus blocks lumen of the blood vessel, resulting in that the blood at the distal end is blocked; 2) thrombus detachment further leads to serious hazards such as pulmonary embolism, cerebral embolism, and myocardial infarction. Thrombus aspiration is a method of sending a catheter to the position of the thrombus under a negative pressure to directly suck the thrombus into the catheter to remove the thrombus.

The advantages of catheter intervention for thrombus aspiration are as follows:
(1) Minimally invasive. Patients with high-risk embolism are in critical condition and often cannot tolerate traditional open surgery. Relatively speaking, interventional treatment is performed by catheter aspiration under local anesthesia, which is less invasive and has a faster recovery.
(2) Rapid effectiveness.

However, the common disadvantage of prior thrombus aspiration devices is that hardened and fibrotic thrombi cannot be effectively extracted with relatively low negative pressure, but if the negative pressure is increased, too much blood will be extracted, resulting in clinical problems caused by excessive blood loss.

Patent document CN1 15844488A discloses a thrombus aspiration pump for automatically adjusting a target flow rate and a method thereof, including an aspiration pump main unit, a liquid storage tank, a connecting pipe, and an aspiration catheter; the aspiration pump main unit is connected to the liquid storage tank; the liquid storage tank is connected to one end of the connecting pipe; a proportional clamp valve and a pressure sensor are provided on a flow path of the liquid storage tank connected to the connecting pipe, a first pressure measuring port and a second pressure measuring port of the pressure sensor are located on the flow path of the liquid storage tank connected to the connecting pipe, and the other end of the connecting pipe is connected to the aspiration catheter. However, this patent cannot completely solve prior technical problems, nor can it meet the objectives of the present invention.

### SUMMARY

In view of the defects in the prior art, an objective of the present invention is to provide an intermittent suction thrombus aspiration pump system.

The intermittent suction thrombus aspiration pump system provided by the present invention includes: a suction catheter, a flow resistor, a first pressure sensor, a second pressure sensor, a metering pump, a clamp valve, a collection container, a third pressure sensor, a negative pressure regulating valve, and a negative pressure pump.

The suction catheter, the first pressure sensor, the flow resistor, the second pressure sensor, the clamp valve, the collection container, the third pressure sensor, the negative pressure regulating valve, and the negative pressure pump are connected in sequence.

Two ends of the metering pump are respectively connected to two ends of the clamp valve.

Preferably, the suction catheter is a catheter that enters a human body and performs thrombus aspiration.

Preferably, the flow resistor is located between the first pressure sensor and the second pressure sensor, so that when blood flows, a pressure difference is generated between the first pressure sensor and the second pressure sensor, and the pressure difference is proportional to a blood flow rate, thereby determining the blood flow rate.

Preferably, the third pressure sensor is configured to measure a negative pressure intensity in the collection container, to determine whether the thrombus aspiration pump system reaches a preset target suction negative pressure.

The negative pressure regulating valve is configured to regulate a negative pressure output by the negative pressure pump, and the negative pressure pump is configured to generate the negative pressure for suction and is a negative pressure source.

Preferably, the flow resistor is a straight pipe or a disc-shaped bending pipe.

Preferably, the metering pump generates a liquid flow direction during working and discharges a liquid on one side of the clamp valve to the other side, thereby generating a larger pressure difference.

Preferably, when the thrombus aspiration pump system performs aspiration, a liquid exists from the suction catheter to the collection container, and an intermittent aspiration is achieved by opening and closing the clamp valve; when the clamp valve is closed, the suction catheter is switched off, at this time, the liquid exists in pipelines on two sides of the clamp valve; at this time, the metering pump starts to work: the liquid downstream of the clamp valve is sucked to the upstream of the clamp valve, the liquid upstream of the clamp valve increases, and a pressure also increases accordingly; the liquid downstream of the clamp valve decreases, and a pressure also decreases accordingly, achieving a purpose of increasing the pressure difference; the third pressure sensor is configured to measure the pressure downstream of the clamp valve, and the first pressure sensor is configured to measure the pressure upstream of the clamp valve; the pressure difference between the third pressure sensor and the first pressure sensor represents a magnitude of the pressure difference generated; and as the pressure difference increases, a thrombus aspiration effect is strengthened.

Preferably, the liquid downstream of the clamp valve is sucked to the collection container, or a liquid from an external pressure source is aspirated to the upstream of the clamp valve, to increase the pressure difference between the upstream of the clamp valve and the downstream of the clamp valve.

Preferably, the second pressure sensor is configured to determine a pressure upstream of the clamp valve. When the pressure upstream of the clamp valve reaches a set limit, the metering pump is stopped.

After the metering pump stops working, the clamp valve is released, and a passage of the suction catheter is restored; and at this time, the pressure difference between the pressure upstream of the clamp valve and the pressure downstream of the clamp valve is instantly released, to achieve a purpose of aspirating a stubborn thrombus.

Preferably, the pressure sensor is replaced with a flow sensor or other sensor or device that directly or indirectly measures a blood flow rate.

Compared with the prior art, the present invention has the following advantages:
(1) The present invention can establish an on-off mechanism on the passage of the aspiration catheter, and control the switch of the on-off mechanism to be on and off at a certain frequency to form the intermittent aspiration. When the suction passage is switched off, a diaphragm pump or the metering pump is used to quickly aspirate the liquid at an end near the collection container to an end near the patient to reduce the pressure at the end near the collection container and increase the pressure at the end near the patient, thereby forming a certain pressure difference. When the pressure difference reaches a certain amount, the suction passage is switched on, and the pressure difference formed at this time can quickly and effectively aspirate the stubborn thrombus.
(2) The present invention can actively increase the pressure difference without increasing the negative pressure output by the negative pressure pump, thereby achieving the purpose of instantly sucking thrombus. If the negative pressure output by the negative pressure pump is increased, the patient may lose too much blood. The actively formed pressure difference only works for a moment and does not increase the patient's blood loss but can effectively suck thrombus. This method can be combined with the intermittent aspiration frequency to achieve the purpose of intermittent aspiration with high frequency and high-pressure difference.

### BRIEF DESCRIPTION OF THE DRAWINGS

By reading the detailed description of the non-limiting embodiments with reference to the following drawings, other features, purposes and advantages of the present invention will become more apparent:
FIG. 1 is a schematic diagram of the structure of an intermittent suction thrombus aspiration pump system of the present invention;
FIG. 2 is a schematic diagram of the structure of the intermittent suction thrombus aspiration pump system of the present invention; and
FIG. 3 is a schematic diagram of the structure of the intermittent suction thrombus aspiration pump system of the present invention.

### DETAILED DESCRIPTION OF THE EMBODIMENTS

The present invention is described in detail below in conjunction with specific embodiments. The following embodiments will help those skilled in the art to further understand the present invention, but do not limit the present invention in any form. It should be noted that for those skilled in the art, several changes and improvements can be made without departing from the concept of the present invention. These all belong to the scope of protection of the present invention.

### Embodiment

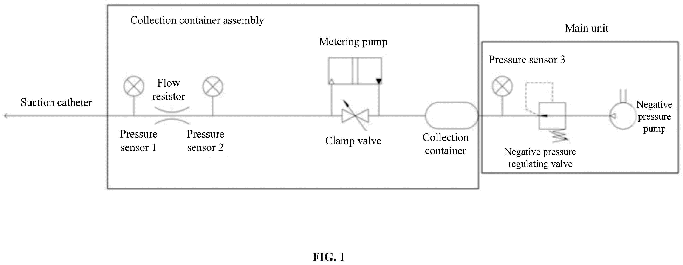

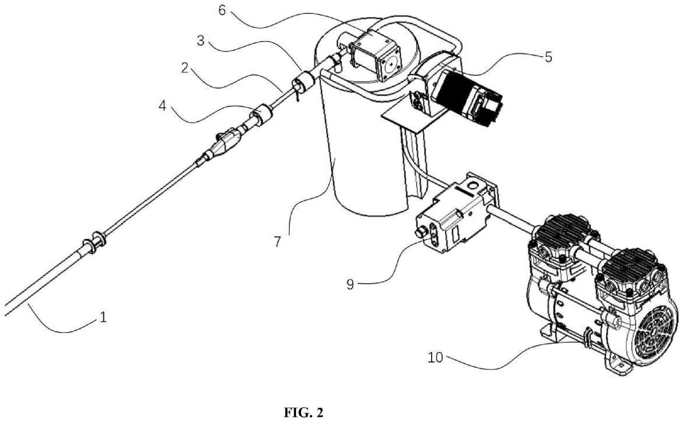

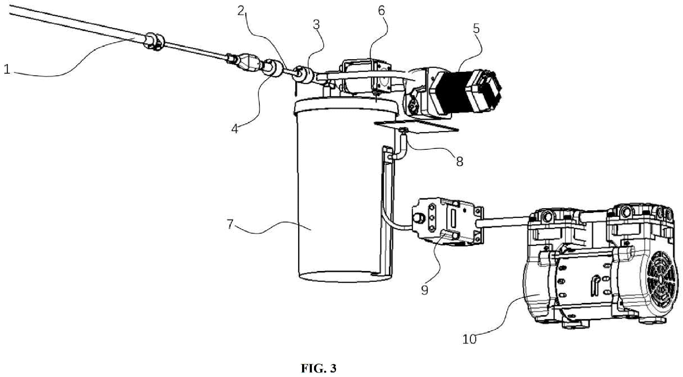

As shown in FIGS. 1-3, the present invention provides an intermittent suction thrombus aspiration pump system, including: the suction catheter 1, the flow resistor 2, the first pressure sensor 4, the second pressure sensor 3, the metering pump 5, the clamp valve 6, the collection container 7, the third pressure sensor 8, the negative pressure regulating valve 9, and the negative pressure pump 10.

The flow resistor 2 is located between the first pressure sensor 4 and the second pressure sensor 3, so that when blood flows, the pressure difference is generated between the first pressure sensor 4 and the second pressure sensor 3, and this pressure difference is proportional to a blood flow rate, thereby determining the blood flow rate.

The third pressure sensor 8 is configured to measure the negative pressure intensity in the collection container 7, to determine whether the system reaches a target suction negative pressure.

The negative pressure regulating valve 9 is configured to regulate a negative pressure output by the negative pressure pump.

The negative pressure pump 10 is configured to generate the negative pressure for suction and is a negative pressure source.

The suction catheter 1 is the catheter that enters the human body and performs thrombus aspiration and is a medical device.

The flow resistor 2 can be a straight pipe, a disc-shaped bending pipe, or other shapes that can generate the pressure difference.

The metering pump 5 can generate a liquid flow direction during working, the purpose of which is to discharge the liquid on one side of the clamp valve 6 to the other side, thereby generating the larger pressure difference.

### Working principle:

When the system performs aspiration, the liquid exists from the suction catheter 1 to the collection container 7, and the intermittent aspiration is achieved by opening and closing the clamp valve 6.

When the clamp valve 6 is closed, the suction catheter 1 is switched off. At this time, the liquid exists in the pipelines on two sides of the clamp valve 6.

At this time, the metering pump 5 starts to work: the liquid downstream of the clamp valve 6 is sucked to the upstream of the clamp valve 6, the liquid upstream of the clamp valve 6 increases, and the pressure also increases accordingly; the liquid downstream of the clamp valve 6 decreases, and the pressure also decreases accordingly, achieving the purpose of increasing the pressure difference. The third pressure sensor 8 is configured to measure the pressure downstream of the clamp valve 6, and the first pressure sensor 4 is configured to measure the pressure upstream of the clamp valve 6. The pressure difference between the third pressure sensor 8 and the first pressure sensor 4 represents the magnitude of the pressure difference generated. As the pressure difference increases, a thrombus aspiration effect is strengthened. **In** practical applications, the pressure difference can often reach two atmospheres, while ordinary aspirations are limited to the negative pressure limit of one atmosphere, so using the liquid aspiration at two ends of the clamp valve 6 can achieve a stronger aspiration effect. The present invention is not limited to aspirating the liquid downstream of the clamp valve 6 to the upstream of the clamp valve 6. The liquid downstream of the clamp valve 6 can also be sucked to the collection container 7, or the liquid from an external pressure source is aspirated to the upstream of the clamp valve 6, to increase the pressure difference between the upstream of the clamp valve 6 and the downstream of the clamp valve 6.

The second pressure sensor 3 is configured to determine the pressure limit upstream of the clamp valve 6. When the pressure upstream of the clamp valve 6 reaches the set limit, the metering pump 5 can be stopped to ensure that the pressure upstream of the clamp valve 6 is not excessively high.

After the metering pump 5 stops working, the clamp valve 6 is released, and the passage of the suction catheter 1 is restored. At this time, the pressure difference between the pressure upstream of the clamp valve 6 and the pressure downstream of the clamp valve 6 formed in the previous step is instantly released, to achieve the purpose of aspirating the stubborn thrombus.

The pressure sensor can also be replaced with a flow sensor or other sensor or device that directly or indirectly measures the blood flow rate.

In the description of the present application, it should be understood that the terms "upper", "lower", "front", "back", "left", "right", "vertical", "horizontal", "top", "bottom", "inside", "outside" and the like indicate the orientation or position relationship based on the orientation or position relationship shown in the drawings, are only used for the convenience of describing the present application and simplifying the description, and do not indicate or imply that the device or element referred to must have a specific orientation, be constructed and operated in a specific orientation, and therefore cannot be understood as a limitation on the present application.

Those skilled in the art know that in addition to implementing the system, device, and its various modules provided by the present invention in a purely computer-readable program code, the system, device, and its various modules provided by the present invention can be implemented in the form of logic gate, switch, application-specific integrated circuit, programmable logic controller, and embedded microcontroller by logically programming the method step to achieve the same program. Therefore, the system, device, and its various modules provided by the present invention can be regarded as the hardware component, and the modules included therein for implementing various programs can also be regarded as structures within the hardware component; the modules for implementing various functions can also be regarded as both software programs for implementing the method and structures within the hardware component.

The above describes the specific embodiments of the present invention. It should be understood that the present invention is not limited to the above specific embodiments, and those skilled in the art can make various changes or modifications within the scope of the claims, which does not affect the essence of the present invention. In the absence of conflict, the embodiments of the present application and the features in the embodiments can be combined with each other arbitrarily.

## Claims

1. An intermittent suction thrombus aspiration pump system, being **characterized in that**: a suction catheter(1), a flow resistor(2), a first pressure sensor(4), a second pressure sensor(3), a metering pump(5), a clamp valve(6), a collection container(7), a third pressure sensor(8), a negative pressure regulating valve(9) and a negative pressure pump(10);
wherein the suction catheter(1), the first pressure sensor(4), the flow resistor(2), the second pressure sensor(3), the clamp valve(6), the collection container(7), the third pressure sensor(8), the negative pressure regulating valve(9) and the negative pressure pump(10) are connected in sequence; and
two ends of the metering pump(5) are respectively connected to two ends of the clamp valve(6).

2. The intermittent suction thrombus aspiration pump system according to claim 1, wherein the suction catheter is configured to enter a human body and perform thrombus aspiration.

3. The intermittent suction thrombus aspiration pump system according to claim 1, wherein the flow resistor(2) is located between the first pressure sensor(4) and the second pressure sensor(3), so that when blood flows, a pressure difference is generated between the first pressure sensor(4) and the second pressure sensor(3), and the pressure difference is proportional to a blood flow rate, thereby determining the blood flow rate.

4. The intermittent suction thrombus aspiration pump system according to claim 1, wherein the third pressure sensor(8) is configured to measure a negative pressure intensity in the collection container(7), to determine whether the intermittent suction thrombus aspiration pump system reaches a preset target suction negative pressure; and
the negative pressure regulating valve(9) is configured to regulate a negative pressure output by the negative pressure pump, and the negative pressure pump(10) is configured to generate the negative pressure for suction and is a negative pressure source.

5. The intermittent suction thrombus aspiration pump system according to claim 1, wherein the flow resistor(2) is a straight pipe or a disc-shaped bending pipe.

6. The intermittent suction thrombus aspiration pump system according to claim 1, wherein
the metering pump(5) generates a liquid flow direction during working and discharges a liquid on a first side of the clamp valve(6) to a second side of the clamp valve(6), thereby generating a larger pressure difference.

7. The intermittent suction thrombus aspiration pump system according to claim 1, wherein
when the intermittent suction thrombus aspiration pump system performs aspiration, the liquid exists from the suction catheter(1) to the collection container(7), and an intermittent aspiration is achieved by opening and closing the clamp valve(6);
when the clamp valve(6) is closed, the suction catheter(1) is switched off, at this time, the liquid exists in pipelines on two sides of the clamp valve(6); at this time, the metering pump(5) starts to work:
the liquid downstream of the clamp valve(6) is sucked to an upstream of the clamp valve(6), the liquid upstream of the clamp valve(6) increases, and a first pressure also increases accordingly; the liquid downstream of the clamp valve(6) decreases, and a second pressure also decreases accordingly, achieving a purpose of increasing the pressure difference;
the third pressure sensor(8) is configured to measure the second pressure downstream of the clamp valve(6), and the first pressure sensor(4) is configured to measure the first pressure upstream of the clamp valve(6); the pressure difference between the third pressure sensor(8) and the first pressure sensor(4) represents a magnitude of the pressure difference generated; and as the pressure difference increases, a thrombus aspiration effect is strengthened.

8. The intermittent suction thrombus aspiration pump system according to claim 1, wherein the liquid downstream of the clamp valve(6) is sucked to the collection container(7), or the liquid from an external pressure source is aspirated to the upstream of the clamp valve(6), to increase the pressure difference between the first pressure upstream of the clamp valve(6) and the second pressure downstream of the clamp valve(6).

9. The intermittent suction thrombus aspiration pump system according to claim 1, wherein the second pressure sensor(3) is configured to determine a third pressure upstream of the clamp valve(6); and when the third pressure upstream of the clamp valve(6) reaches a set limit, the metering pump(5) is stopped; and
after the metering pump(5) stops working, the clamp valve(6) is released, and a passage of the suction catheter(1) is restored; and at this time, the pressure difference between the first pressure upstream of the clamp valve(6) and the second pressure downstream of the clamp valve(6) is instantly released, to achieve a purpose of aspirating a stubborn thrombus.

10. The intermittent suction thrombus aspiration pump system according to claim 1, wherein the first pressure sensor(4), the second pressure sensor(3) or the third pressure sensor(8) is replaced with a flow sensor or a sensor or a device, wherein the sensor or the device directly or indirectly measures the blood flow rate.
